Europäisches Patentamt

European Patent Office

Office européen des brevets

(19)

(11) Numéro de publication : **0 422 719 B1**

(12) **FASCICULE DE BREVET EUROPEEN**

(45) Date de publication du fascicule du brevet :
08.12.93 Bulletin 93/49

(51) Int. Cl.⁵ : **A61L 27/00, A61K 6/033, C01B 15/16**

(21) Numéro de dépôt : **90202599.8**

(22) Date de dépôt : **28.09.90**

(54) **Biomatériau de comblement osseux ou dentaire, et procédé de préparation.**

(30) Priorité : **09.10.89 FR 8913246**

(43) Date de publication de la demande :
**17.04.91 Bulletin 91/16**

(45) Mention de la délivrance du brevet :
**08.12.93 Bulletin 93/49**

(84) Etats contractants désignés :
**BE DE ES FR GB IT SE**

(56) Documents cités :
**EP-A- 0 166 263
EP-A- 0 214 070
EP-A- 0 284 074
EP-A- 0 324 425
JOURNAL OF INORGANIC & NUCLEAR CHE-
MISTRY, vol. 40, no. 1, 1978, pages 27-30,
Pergamon Press, GB; C. REY et al.: "Some
features of the incorporation of oxygen in
different oxidation states in the apatitic lattice
- III"**

(73) Titulaire : **AGENCE CONSEIL 3 P
143 rue Anatole France
F-92300 Levallois Perret (FR)**

(72) Inventeur : **Ledard, Claude
8 boulevard Lelasseur
F-44000 Nantes (FR)**
Inventeur : **Benque, Edmond
63 boulevard Lazare Carnot
F-31000 Toulouse (FR)**
Inventeur : **Lacout, Jean-Louis
36 rue de la Colombette
F-31000 Toulouse (FR)**
Inventeur : **Rey, Christian
Bois des Dames Aureville
F-31320 Castenet Tolosan (FR)**

(74) Mandataire : **Barre, Philippe
Cabinet Barre-Gatti-Laforgue 95 rue des
Amidonniers
F-31069 Toulouse Cédex (FR)**

EP 0 422 719 B1

## Description

L'invention concerne un biomatériau de comblement osseux ou dentaire. Elle vise un biomatériau de comblement apte à favoriser une réhabitation par le tissu osseux ou dentaire et à permettre une substitution partielle ou totale du biomatériau par le tissu. L'invention s'étend à des procédés de fabrication dudit biomatériau.

On connaît des biomatériaux de comblement intraosseux ou intradentaires à base de phosphate de calcium et en particulier à base h'hydroxyapatite $Ca_{10}$ $(PO_4)_6$ $(OH)_2$. On pourra par exemple se reporter aux publications suivantes concernant ces biomatériaux apatitiques : "Jarcho M, Kay J.F, Gumaer KI, Doremus R.H and Drobeck H.P Tissue, cellular and subcellular events at a bone-ceramic hydroxylapatite interface. J. Bioeng 1, 79, 1977"; "Ducheyne P and de Groot K, In vivo surface activity of a hydroxyapatite alveolar bone substitute, J. Biomed. Mater. Res. 14, 225, 1980" ; "Klaas de Groot, Bioceramics of calcium phosphate, C.R.S. Press Inc., New-York, 1983".

Ces biomatériaux sont connus pour présenter les propriétés suivantes :
- excellente biocompatibilité (risques très limités de réactions de rejets inflammatoires),
- très faible solubilité (faible tendance à disparaître dans le milieu au cours des quelques mois succédant l'implantation),
- qualité d'ostéoinduction (tendance à favoriser la reconstruction osseuse).

Ces propriétés qui rendent ce matériau très approprié à la réalisation de comblements osseux ou dentaires, proviennent de leur composition chimique et de leur structure cristallographique, qui sont voisines de celles de la partie minérale des tissus calcifiés.

Toutefois, ces biomatériaux apatitiques connus sont inactifs vis-à-vis des micro-organismes pathogènes susceptibles de se développer dans les tissus. Ainsi, ces biomatériaux ont essentiellement un rôle mécanique (remplissage et substitution osseuse) et n'ont aucune propriété antiseptique : ils sont totalement inaptes à limiter le développement des bactéries, champignons... qui sont présents avant l'intervention (caries, maladies paradontales...) ou qui en sont la conséquence (asepties imparfaites notamment en bouche, inflammations...).

La présente invention se propose de fournir un nouveau biomatériau présentant, comme les matériaux connus précités, une structure apatitique lui conférant d'excellentes qualités de biocompatibilité et d'ostéoconduction ou induction, mais qui présente des propriétés antiseptiques le rendant capable de limiter la prolifération des micro-organismes dans le site d'implantation.

L'invention vise ainsi à protéger l'allogreffe de façon locale au niveau du site d'implantation, en évitant une utilisation d'antibiotiques par voie générale. Elle vise également à assurer cette protection d'une façon continue dans le temps, pendant toute la durée où le matériau reste présent dans le site d'implantation.

Un autre objectif de l'invention est de permettre, suivant le cas traité, d'adapter le champ d'action du biomatériau au caractère des micro-organismes présents ou susceptibles de se développer sur le site d'implantation (en particulier en vue de lutter de façon spécifique contre les micro-organismes anaérobies).

A cet effet, le biomatériau de comblement osseux ou dentaire conforme à l'invention comprend du phosphate de calcium oxygéné (ApO) ayant une structure apatitique possédant des expèces oxygénées à des degrés d'oxydation supérieurs ou égaux à -2, de formule :
$$Ca_{10-x} \square_x (HPO_4)_y (PO_4)_{6-y} (OH)_z (O_2^{2-})_t \square_{2-z-t} (O_2)_u$$
où $\square$ représente une lacune,
$$0 \leqq x \leqq 1$$
$$0 \leqq y \leqq 1$$
$$0 \leqq t \leqq 1$$
$$0 \leqq u \leqq 1$$
$$u + t \geqq 0,2$$
et $z = 2 + y - x - 2t$

Un tel biomatériau contient dans les tunnels de sa structure apatitique au moins l'une des deux espèces suivantes : $O_2^{2-}$, $O_2$. Les expérimentations ont montré que ces espèces étaient libérées dans le milieu soit par dissolution progressive du matériau, soit par échange chimique avec le milieu. L'espèce $O_2^{2-}$ (ion peroxyde) ainsi libérée agit in situ pour détruire les micro-organismes avec une efficacité bien connue en soi pour cette espèce ; l'espèce $O_2$ (oxygène moléculaire) agit de façon spécifique sur les micro-organismes anaérobies en augmentant localement la pression partielle d'oxygène. Tout en conservant une structure apatitique et les propriétés afférentes de biocompatibilité et d'ostéoconduction ou induction le biomatériau conforme à l'invention engendre donc un effet de protection du site d'implantation à l'égard de toute apparition ou développement d'organismes pathogènes. La vitesse de dissolution dans le milieu et donc la durée d'efficacité du matériau peuvent être ajustées en faisant varier la stoechiométrie de la structure apatitique et notamment les coefficients x et y, le nombre de lacunes en site calcique et d'ions $HPO_4$ étant directement lié à la vitesse de dissolution (matériau peu soluble pour x < 0,1, y < 0,1 et de solubilité croissante pour x > 0,1 , y > 0,1).

L'invention vise en particulier trois sous-familles du biomatériau précédemment défini.

Le biomatériau de la première sous-famille contient, à la fois, les espèces $O_2^{2-}$ et $O_2$ de façon à présenter une action antiseptique générale sur les micro-organismes (présence de $O_2^{2-}$), renforcée vis-à-vis des anaérobies (présence de $O_2$). Le biomatériau de cette sous-famille comprend un phosphate de calcium oxygéné (ApO) de structure apatitique dans laquelle les coefficients sont tels que :

$0,1 \leq x \leq 0,4$

y peu différent de x

$t \leq \dfrac{u}{2}$

$0,2 \leq u \leq 0,8$

Il est à noter que la solubilité de ce biomatériau peut être ajustée dans une large plage depuis une faible solubilité (x de l'ordre de 0,1) jusqu'à une solubilité élevée (x de l'ordre de 0,4).

Le biomatériau de la deuxième sous-famille contient essentiellement l'espèce $O_2^{2-}$ et simplement des traces de l'espèce $O_2$. Ce matériau très riche en groupement peroxyde présente une action générale antiseptique très efficace. Le biomatériau de cette sous-famille comprend un phosphate de calcium oxygéné (ApO) de structure apatitique dans laquelle les coefficients sont tels que :

$0,2 \leq x \leq 0,5$

y peu différent de x

$0,8 \leq t \leq 1$

$u < 0,1$

Ce matériau (issu comme on le verra d'une fabrication à basse température) possède un coefficient x supérieur à 0,2 (nombre de lacune relativement élevé) et présente donc une solubilité généralement plus importante que le matériau précédent.

Le biomatériau de la troisième sous-famille contient exclusivement l'espèce $O_2$ de sorte qu'il présente une action sélective très efficace vis à vis des micro-organismes anaérobies. Ce biomatériau est important en pratique car ce sont généralement des micro-organismes de ce type qui se développent en milieu d'implantation; en outre, dans certains cas, il est préférable d'eviter l'action générale de destruction de l'espèce $O_2^{2-}$, en particulier pour préserver certains micro-organismes qui développent une action favorable. Le biomatériau de cette troisième sous-famille comprend un phosphate de calcium oxygéné (ApO) de structure apatitique suivante :

$Ca_{10-} \square_x (H\,PO_4)_y (PO_4)_{6-y} (OH)_{2+y-x} \square_{1-y} (O_2)_u$

où

$0 \leq x \leq 0,2$

$0 \leq y \leq 0,2$

$0,2 \leq u \leq 0,8$

La solubilité de ce biomatériau est généralement plus faible que celle des précédents, mais peut être ajustée à une valeur parfaitement satisfaisante pour assurer une pression locale d'oxygène suffisante. Il est à noter que, généralement, la présence de micro-organismes pathogènes engendre une baisse locale du pH qui augmente la dissolution du biomatériau et donc le dégagement d'oxygène au moment du besoin.

Le biomatériau de l'invention peut être utilisé, notamment dans le cas où les espèces actives $O_2^{2-}$ et $O_2$ sont en faible teneur (t et u faibles) ou lorsque l'on se trouve sur un site à haut risque inflammatoire (intervention sur carie ou maladie osseuse telle qu'ostéomyélite). Il peut également être utilisé mélangé à d'autres matériaux de comblement connus afin d'associer les qualités propres de ces matériaux à celles antiseptiques du biomatériau conforme à l'invention ; les additifs peuvent en particulier être les suivants : phosphate de calcium apatitique non oxygéné, encore désigné par hydroxyapatite HAp de formule $Ca_{10}(PO_4)_6(OH)_2$ (caractéristiques : très faible solubilité, bonne tenue mécanique et porosité élevée donnant de bonnes qualités d'ostéoconduction) ; phosphate tricalcique PTCa de formule $Ca_3(PO_4)_2$ (généralement à faible teneur en raison de sa forte solubilité) ; carbonate de calcium $CaCO_3$ (naturel ou synthétique) et/ou sulfate de calcium $CaSO_4$ (faible coût et prise avec expansion) ; dérivés sanguins de coagulation, en particulier fibrine, fibrinogène... ; collagène (malléabilité et adhérence) éventuellement complété de glycoaminoglycanes et de sulfate de chondroitine (action ostéoinductrice).

De plus, il est possible de substituer partiellement des ions hydrogénophosphates ($HPO_4$) par des ions carbonates ($CO_3$) dans la structure apatitique oxygénée du biomatériau conforme à l'invention. Cette substitution rapproche encore la composition du biomatériau de celle de la partie minérale de l'os, tout en conservant le caractère antiseptique au biomatériau.

L'invention s'étend à des procédés de fabrication du biomatériau conforme à l'invention permettant d'obtenir l'apatite oxygénée appropriée à chaque application, procédés permettant en particulier de fabriquer des biomatériaux conformes aux trois sous-familles définies précédemment.

Il est à noter que de l'apatite oxygénée a déjà été obtenue en laboratoire par Dale R. SIMPSON et 1969 ("D.R. SIMPSON, oxygen rich apatite, The Am. Min. vol. 54 p. 560-562, March-April 69"). D. SIMPSON a montré que ce composé était soluble dans l'acide chlorhydrique et dégageait de l'oxygène apte à oxyder à chaud une électrode en cuivre. Toutefois, cette fabrication ponctuelle de laboratoire ne permet pas d'ajuster les coefficients des diverses espèces et d'obtenir le corps que l'on désire.

Selon un premier mode de préparation conforme à l'invention, le procédé consiste à mettre en suspension un phosphate calcique du groupe suivant : phosphate tricalcique (PTCa), phosphate dicalcique (DCPD), ou phosphate octocalcique (OCP), dans une solution aqueuse contenant de l'eau oxygénée, à laisser évoluer la suspension à un pH, à une température et pendant une durée fonctions des coefficients x, y, t et u désirés, à séparer physiquement la phase solide et la phase liquide et à sécher ladite phase solide.

Selon un autre mode de préparation, le procédé consiste à verser dans une solution aqueuse contenant de l'eau oxygénée, d'une part un sel soluble de calcium, d'autre part du phosphate d'ammonium, à un pH, à une température et pendant une durée fonctions des coefficients x, y, t et u désirés, à laisser maturer le précipité obtenu dans la solution-mère, à séparer physiquement la phase solide et la phase liquide et à sécher ladite phase solide.

Le second mode de préparation sera de préférence choisi pour favoriser la teneur en ions péroxyde du biomatériau.

Pour optimiser cette préparation, et éviter la formation de phases étrangères et des pertes en réactifs, les quantités de sels de calcium et de phosphate d'ammonium versées dans la solution aqueuse d'eau oxygénée sont telles que le rapport atomique $\frac{Ca}{P}$ soit compris entre 1,4 et 2.

Dans les deux modes de préparation, le pH lors de l'évolution ou de la précipitation est avantageusement fixé à une valeur comprise entre 5 et 9, d'autant plus élevée que l'on désire des coefficients x et y plus faibles dans les plages $0 \le x \le 1$ et $0 \le y \le 1$. De préférence le pH est ajusté par ajout d'ammoniaque, l'ion ammonium étant peu gênant et facilement éliminable.

La température lors de l'évolution ou de la précipitation est avantageusement fixée à une valeur comprise entre 20° C et 100° C, d'autant plus élevée que l'on désire des coefficients x, y et t plus faibles dans les plages $0 \le x \le 1$, $0 \le y \le 1$ et $0 \le t \le 1$.

La durée pendant laquelle on laisse évoluer la suspension ou pendant laquelle s'effectue la précipitation est un facteur important pour obtenir, à la fois, une bonne cristallisation de l'apatite et des valeurs désirées des coefficients x, y et t.

Cette durée est avantageusement fixée à une valeur comprise entre 2 minutes et 300 heures, d'autant plus longue que l'on désire des coefficients x, y et t plus faibles dans les plages $0 \le x \le 1$ et $0 \le y \le 1$ et $0 \le t \le 1$.

Dans le cas de la préparation par précipitation, le versement des sels de calcium et de phosphore s'effectue progressivement dans la solution d'eau oxygénée pendant la durée sus-évoquée. Pour les longues durées, ce versement est fait en goutte à goutte avec un débit approprié.

La concentration en eau oxygénée de la solution aqueuse joue sur les teneurs en $O_2^{2-}$ et en $O_2$. De préférence, on choisit une solution aqueuse d'eau oxygénée de concentration comprise entre 5 et 50 volumes, d'autant plus élevée que l'on désire des coefficients t et u plus élevés dans les plages $0 \le t \le 1$ et $0 \le u \le 1$.

Quel que soit le mode de mise en oeuvre, le procédé de préparation peut être complété par une étape supplémentaire de calcination de la phase solide à une température inférieure à 450° C, d'autant plus élevée que l'on désire un coefficient t plus faible dans la plage $0 \le t \le 1$.

Cette calcination modifie la composition du biomatériau en transformant l'espèce péroxyde $O_2^{2-}$ en oxygène moléculaire $O_2$. La calcination réduit donc le coefficient t au profit du coefficient u, mais on observe toutefois une perte globale d'oxygène, la somme t + u obtenue étant inférieure à la somme t + u initiale. De plus, cette calcination améliore l'état de cristallisation et réduit la solubilité du produit. Comme elle est effectuée à des températures moyennes, la solubilité finale du biomatériau calciné demeure toutefois suffisante pour lui conférer de bonnes propriétés antiseptiques dans le site d'implantation.

Pour l'obtention d'un biomatériau constitué par une association d'apatite oxygénée et d'un ou de matériaux supplémentaires du groupe évoqué précédemment, il suffit de mélanger par voie physique et de façon homogène la phase solide sèche de ApO obtenue avec le ou les composés désirés se présentant sous forme divisée ou sous forme de gel aqueux : HAp, PTCa, $CaCO_3$, $CaSO_4$, collagène, glycoaminoglycane, sulfate de chondroitine.

Dans le cas d'un gel aqueux de collagène, le mélange peut ensuite être lyophilisé afin d'obtenir une poudre sèche prête à l'emploi.

Pour préparer le biomatériau déjà défini dans lequel des ions hydrogénophosphates sont substitués par des ions carbonates, on ajoute dans la solution contenant l'eau oxygénée du carbonate de potassium ou de sodium et le procédé est ensuite mis en oeuvre comme déjà défini. En pratique, cet ajout demeure très faible par rapport à la quantité des autres composants (rapport atomique $\frac{CO_3}{Ca} < 0,05$) afin d'obtenir une substitution très minoritaire.

La description qui suit fournit des exemples de préparation de biomatériaux conformes à l'invention et des résultats d'essais.

Les méthodes d'analyses utilisées sont dans tous les cas des méthodes classiques de la chimie du solide :

- diffraction des rayons X : la diffraction des rayons X permet de mettre en évidence la structure cristallographique en effectuant le dénombrement, la classification et la détermination des positions des raies. Un examen plus poussé permet en outre de définir les paramètres cristallins de la maille, et de donner une idée (largeur de raies) de l'état de cristallisation. Enfin, la diffraction des rayons X permet de s'assurer, à la sensibilité de la méthode, de la pureté du composé,

- spectrométrie d'absorption infrarouge : la spectrométrie d'absorption infrarouge permet de mettre en évidence les liaisons dans des groupements atomiques. Dans le cas des apatites on peut en particulier mettre en évidence :
  . les ions hydroxydes : bandes à 3560 et 740 cm$^{-1}$,
  . les ions carbonates domaine 1420 cm$^{-1}$,

. les bandes phosphate et plus particulièrement la bande due aux ions $HPO_4^=$,

- détermination du phosphore : la détermination du phosphore (ions orthophosphate) est effectuée par analyse chimique par une méthode colorimétrique : formation du complexe phosphomolybdique jaune. Par cette méthode on détermine l'ensemble des ions orthophosphate (dans notre cas $PO_4^{3-}$ et $HPO_4^=$),

- détermination des ions $HPO_4^=$ : la détermination des seuls ions $HPO_4^=$ est effectuée par analyse avant et après calcination à 1000° C. Le dosage avant calcination permet de doser l'ensemble des ions $PO_4^{3-}$ et $HPO_4^=$. La calcination transforme les ions $HPO_4^=$ en ions $P_2O_7^{4-}$. Le dosage après calcination et sans hydrolyse ne dose pas les ions $P_2O_7^{4-}$. La différence entre le dosage avant et après calcination permet d'obtenir la valeur correspondant aux seuls ions $HPO_4^=$,

- détermination de l'oxygène moléculaire : la détermination de l'oxygène moléculaire s'effectue par mesure du volume dégagé lors de la dissolution acide ; en présence de carbonate on doit préalablement absorber le $CO_2$ dégagé sur de l'amiante sodée,

- détermination des ions $O_2^=$ : après dissolution acide (acide perchlorique 1 N) on dose les ions $O_2^=$ par manganimétrie.

EXEMPLE 1 : PREPARATION DE BIOMATERIAU

On prépare une solution de 1 litre d'eau oxygénée à 30 volumes par dilution d'une solution commerciale à 110 volumes et on dissout dans cette solution 2,6 grammes de phosphate diammonique $(NH_4)_2HPO_4$ (solution 1). On prépare une solution de 100 millilitres d'eau contenant 7,08 grammes de nitrate de calcium $(Ca(NO_3)_2,6H_2O)$ (solution 2). On porte la solution 1 à la température de 90° C et on amène le pH à la valeur de 8,5 par ajout de la quantité nécessaire d'ammoniaque. On verse la solution 2 dans la solution 1 en une seule fois ; on laisse une heure à la température de 90° C en maintenant le pH à la valeur de consigne 8,5. On sépare le précipité de sa solution mère par filtration sur buchner et on sèche le solide recueilli.

Tous les réactifs sont de qualité pharmaceutique. Cette préparation permet de préparer environ 3,5 grammes de biomatériau.

Le biomatériau est une apatite de formule :
$$Ca_{9,3}(PO_4)_5(HPO_4)(O_2^=)_{0,85}(OH)(O_2)_{0,05}$$

EXEMPLE DE PREPARATION N°2 :

On prépare une solution de 1 litre d'eau oxygénée à 30 volumes par dilution d'une solution commerciale à 110 volumes et on en fixe le pH à la valeur 8,5 par ajout d'une quantité suffisante d'ammoniaque diluée (au dixième). 50 grammes de phosphate tricalcique β $(Ca_3(PO_4)_2)$ finement broyés sont ajoutés à cette solution. Cette suspension est agitée lentement au moyen d'un agitateur magnétique et chauffée à reflux pendant 10 heures à une température proche de l'ébullition. Une électrode de mesure du pH, supportant la température (type Métrohm), permet de contrôler le pH en permanence : en cas de variation il est ramené à la valeur de consigne 8,5 par ajout d'ammoniaque diluée au dixième. On laisse ensuite refroidir ; on sépare le solide de la solution par filtration sur buchner et on lave avec 2 litres d'eau distillée jusqu'à éliminer tous les sels en solution. On sèche le solide dans une étuve à 30° C pendant 5 heures, puis on le chauffe à l'air pendant 1 heure à 430° C. On obtient ainsi environ 45 grammes d'un solide pulvérulent ; il est caractérisé par diffraction des rayons X, spectrométrie d'absorption infrarouge et analyse chimique. Il répond à la formule :
$$Ca_{9,9}(PO_4)_{5,9}(HPO_4)_{0,1}(OH)(O_2)_{0,5}$$

EXEMPLE 3 : PREPARATION D'UNE PATE DE COMBLEMENT CANALAIRE

Une première préparation très simple a été réalisée ; elle consiste à mélanger soigneusement : 0,5 gramme d'apatite oxygénée préparée selon l'exemple n° 1, avec 0,5 gramme d'hydroxyapatite stoichiométrique et 0,1 gramme de sulfate de calcium anhydre $(CaSO_4)$. Chacun des différents constituants est préalablement broyé (environ 5 microns) ; le mélange est également soigneusement homogénéisé. Il peut être conservé en flacon hermétiquement bouché. Au moment de l'emploi le mélange est rendu pâteux et amené à la consistance voulue par adjonction d'eau distillée (environ 1 ml pour 50 mg). Cette pâte peut être alors utilisée pour l'obturation canalaire au moyen des instruments professionnels couramment employés dans ce cas.

EXEMPLE 4 DE PREPARATION D'UNE PATE

La préparation est analogue à celle précédemment décrite ; on remplace simplement l'ajout d'eau distillée par l'ajout d'une solution à 0,5 % de collagène natif de veau.

EXEMPLE 5 DE PREPARATION DE GRANULES

Selon la méthode de préparation de l'exemple n° 2 on peut également préparer des granulés. Pour cela après lavage et filtration le solide est récupéré, séché à l'étuve à 80° C jusqu'à ce qu'il soit amené à une consistance convenant à la granulation (pourcentage en eau voisin de 10 %). Il est ensuite granulé dans une machine de type "FREWITT MG 6A". Le tamis supérieur est fixé à 400 microns et la coupure inférieure à 200 microns. Les granulés sont ensuite calcinés à l'air 1 heure à 430° C.

EXEMPLE 6 : RESULTATS D'ESSAIS IN VITRO

L'efficacité des biomatériaux conformes à l'invention à base d'apatites oxygénées a été testée in vitro sur des bactéries anaérobies. Deux souches ont été retenues :
- bacteroides fragilis (collection Institut Pasteur NCTC 9343),
- fusobacterium nucleatum (collection Institut Pasteur Ref 6039).

Ces deux souches ont été choisies parce qu'elles sont strictement anaérobies et qu'on les retrouve dans différentes pathologies humaines.

Le principe de ces tests in vitro consiste à mettre en présence les bactéries anaérobies avec différentes concentrations d'apatites en suspension (bouillon) dans des conditions contrôlées d'anaérobiose et à déterminer le nombre de ces bactéries après 48 heures et 72 heures de contact. Le témoin est constitué par une apatite ne contenant pas d'espèces oxygénées.

On observe dans les deux cas une réduction importante par un facteur 1000 du nombre de bactéries anaérobies.

EXEMPLE 7: IMPLANTATION IN VIVO

Quelques tests ont été réalisés in vivo sur des chiens aussi bien en obturation canalaire qu'en comblement parodontal. Les implants se comportent de façon analogue aux implants réalisés à partir d'autres hydroxyapatites. On ne note aucun signe de rejet ou d'inflammation.

**Revendications**

1. Biomatériau de comblement osseux ou dentaire, ayant des propriétés antiseptiques en vue de limiter la proiifération des micro-organismes dans le site d'implantation, caractérisé en ce qu'il comprend du phosphate de calcium oxygéné (ApO) ayant une structure apatitique possédant des espèces oxygénées à des degrés d'oxydation supérieurs ou égaux à -2, de formule :
$Ca_{10-x} \square_x (HPO_4) (PO_4)_{6-y} (OH)_z (O_2^{2-})_t \square_{2-z-t} (O_2)_u$
où $\square$ représente une lacune,
$0 \leq x \leq 1$
$0 \leq y \leq 1$
$0 \leq t \leq 1$
$0 \leq u \leq 1$
$u + t \geq 0,2$
et $z = 2 + y - x - 2t$

2. Biomatériau selon la revendication 1, caractérisé en ce qu'il comprend un phosphate de calcium oxygéné (ApO) de structure apatitique dans laquelle les coefficients sont tels que :
$0,1 \leq x \leq 0,4$
y peu différent de x
$t \leq \dfrac{u}{2}$
$0,2 \leq u \leq 0,8$

3. Biomatériau selon la revendication 1, caractérisé en ce qu'il comprend un phosphate de calcium oxygéné (ApO) de structure apatitique dans laquelle les coefficients sont tels que :
$0,2 \leq x \leq 0,5$
y peu différent de x
$0,8 \leq t \leq 1$
$u < 0,1$

4. Biomatériau selon la revendication 1, caractérisé en ce qu'il comprend un phosphate de calcium oxygéné (ApO) de structure apatitique suivante :
$Ca_{10-x} \square_x (H PO_4)_y (PO_4)_{6-y} (OH)_{2+y-x} \square_{1-y} (O_2)_u$
où
$0 \leq x \leq 0,2$
$0 \leq y \leq 0,2$
$0,2 \leq u \leq 0,8$

5. Biomatériau selon l'une des revendications 1, 2, 3 ou 4, caractérisé en ce qu'il comprend un mélange du phosphate de calcium apatitique oxygéné (ApO) de formule précitée et d'un phosphate de calcium apatitique non oxygéné (HAp) de formule : $Ca_{10} (PO_4)_6 (OH)_2$

6. Biomatériau selon l'une des revendications 1, 2, 3 ou 4, caractérisé en ce qu'il comprend un méiange du phosphate de calcium apatitique oxygéné (ApO) de formule précitée et d'un phosphate tricalcique (PTCa) de formule : $Ca_3 (PO_4)_2$.

7. Biomatériau selon l'une des revendications 1, 2, 3 ou 4, caractérisé en ce qu'il comprend un mélange du phosphate de calcium apatitique oxygéné (ApO) de formule précitée et de carbonate de calcium Ca $CO_3$ et/ou sulfate de calcium $CaSO_4$.

8. Biomatériau selon l'une des revendications 1, 2, 3 ou 4, caractérisé en ce qu'il comprend un méiange de phosphate de calcium apatitique oxygéné (ApO) de formuie précitée et d'un coiiagène.

9. Biomatériau selon la revendication 8, caractérisé en ce qu'il comprend un méiange du phosphate de calcium apatitique oxygéné (ApO), de collagène, de glycoaminoglycanes et de sulfate de chondroitine.

10. Biomatériau selon l'une des revendications 1, 2, 3 ou 4, caractérisé en ce qu'il comprend un mélange de phosphate de calcium apatitique oxygéné (ApO) de formule précitée et de dérivés sanguins de coagulation, en particulier fibrine, fibrinogène.

11. Biomatériau selon l'une des revendications précédentes, caractérisé en ce qu'il comprend du phosphate de calcium oxygéné (ApO) de structure apatitique dans laquelle une fraction des ions hydrogénophosphates ($HPO_4$) est substituée par des ions carbonates ($CO_3$).

12. Procédé de préparation d'un biomatériau conforme à l'une des revendications 1, 2, 3 ou 4, caractérisé en ce qu'il consiste à mettre en suspension un phosphate calcique du groupe suivant : phosphate tricalcique (PTCa), phosphate dicalcique (DCPD) ou phosphate octocalcique (OCP), dans une solution aqueuse contenant de l'eau oxygénée, à laisser évoluer la suspension à un pH, à une température et pendant une durée fonctions des coefficients x, y, t et u désirés, à séparer physiquement la phase solide et la phase liquide et à sécher ladite phase solide.

13. Procédé de préparation d'un biomatériau conforme à l'une des revendications 1, 2, 3 ou 4, caractérisé en ce qu'il consiste à verser dans une solution aqueuse contenant de l'eau oxygénée, d'une part un sel soluble de calcium, d'autre part du phosphate d'ammonium, à un pH, à une température et pendant une durée fonctions des coefficients x, y, t et u désirés, à laisser maturer le précipité obtenu dans la solution-mère, à séparer physiquement la phase solide et la phase liquide et à sécher ladite phase solide.

14. Procédé de préparation selon la revendication 13, caractérisé en ce que les quantités de sel de calcium et de phosphate d'ammonium versées dans la solution aqueuse d'eau oxygénée sont telles que le rapport atomique $\frac{Ca}{P}$ soit compris entre 1,4 et 2.

15. Procédé de préparation selon l'une des revendications 12, 13 ou 14, caractérisé en ce que le pH lors de l'évolution ou de la précipitation est fixé à une valeur comprise entre 5 et 9, d'autant plus élevée que l'on désire des coefficients x et y plus faibles dans les plages $0 < x \leqq 1$ et $0 < y \leqq 1$.

16. Procédé de préparation selon l'une des revendications 12, 13, 14 ou 15, caractérisé en ce que la température lors de l'évolution ou de la précipitation est fixée à une valeur comprise entre 20° C et 100° C, d'autant plus élevée que l'on désire des coefficients x, y et t plus faibles dans les plages $0 < x \leqq 1$, $0 < y \leqq 1$ et $0 \leqq t \leqq 1$.

17. Procédé de préparation selon l'une des revendications 12, 13, 14, 15 ou 16, caractérisé en ce que la durée pendant laquelle on laisse évoluer la suspension ou pendant laquelle s'effectue la précipitation est fixée à une valeur comprise entre 2 minutes et 300 heures, d'autant plus longue que l'on désire des coefficients x, y et t plus faibles dans les plages $0 \leqq x \leqq 1$ et $0 \leqq y \leqq 1$ et $0 \leqq t \leqq 1$.

18. Procédé de préparation selon l'une des revendications 12 à 17, caractérisé en ce que la solution aqueuse contenant de l'eau oxygénée est choisie de concentration comprise entre 5 et 50 volumes, d'autant plus élevée que l'on désire des coefficients t et u plus élevés dans les plages $0 \leqq t \leqq 1$ et $0 \leqq u \leqq 1$.

19. Procédé de préparation selon l'une des revendications 12 à 18, caractérisé en ce qu'il comprend une étape supplémentaire de calcination de la phase solide séparée, à une température inférieure à 450°, d'autant plus élevée que l'on désire un coefficient t plus faible dans la plage $0 \leqq t \leqq 1$.

20. Procédé selon la revendication 12 pour préparer un biomatériau conforme à la revendication 2 ayant une teneur réduite d'ions péroxyde $O^{2-}$, caractérisé en ce que, une fois séchée, la phase solide est calcinée à une température comprise entre 150° et 300°, d'autant plus élevée que l'on désire un coefficient t plus faible.

21. Procédé selon la revendication 13 pour préparer un biomatériau conforme à la revendication 3, caractérisé en ce qu'il consiste :
   . à préparer une solution aqueuse d'eau oxygénée de concentration comprise entre 25 et 35 volumes,
   . à verser dans ladite solution aqueuse d'eau oxygénée, d'une part, une solution aqueuse de nitrate ou d'acétate de calcium, d'autre part, une solution aqueuse de phosphate d'ammonium, de façon que le rapport atomique $\frac{Ca}{P}$ soit compris entre 1,5 et 1,7 ,
   . à mélanger les solutions à un pH compris entre 8 et 9, à une température comprise entre 75° C et 100° C et pendant une durée comprise entre 2 et 10 minutes,
   . à séparer, au terme de la durée précitée, la phase solide et à la sécher à une température inférieure à 100° C.

**22.** Procédé de préparation selon l'une des revendications 12 à 21 en vue d'obtenir un biomatériau conforme à l'une des revendications 5 à 9, caractérisé en ce que la phase solide sèche est mélangée avec au moins un composé du groupe suivant, se présentant sous forme divisée ou sous forme de gel aqueux : HAp, PTCa, $CaCO_3$, $CaSO_4$, collagène, glycoaminoglycane, sulfate de chondroitine.

**23.** Procédé de préparation selon l'une des revendications 12 à 22, en vue d'obtenir un biomatériau conforme à la revendication 10, caractérisé en ce que l'on ajoute dans la solution contenant l'eau oxygénée du carbonate de potassium, ou de sodium.

**Claims**

**1.** Biomaterial for osseous or dental filling, having antiseptic properties with a view to limiting the proliferation of micro-organisms at the implantation site, characterised in that it comprises oxygenated calcium phosphate (ApO) of apatitic structure comprising compounds oxygenated to degrees of oxidation higher than or equal to 2-, of the formula :

$$Ca_{10-x} \square_x (HPO_4)_y (PO_4)_{6-y} (OH)_z (O_2^{2-})_t \square_{2-z-t} (O_2)_u$$

where $\square$ corresponds to a gap,

$0 \leqq x \leqq 1$
$0 \leqq y \leqq 1$
$0 \leqq t \leqq 1$
$0 \leqq u \leqq 1$
$u + t \geqq 0.2$
and $z = 2 + y - x - 2t$

**2.** Biomaterial according to Claim 1, characterised in that it comprises an oxygenated calcium phosphate (ApO) of apatitic structure in which the coefficients are such that :

$0.1 \leqq x \leqq 0.4$
$y$ differs little from $x$
$t \leqq u/2$
$0.2 \leqq u \leqq 0.8$

**3.** Biomaterial according to Claim 1, characterised in that it comprises an oxygenated calcium phosphate (ApO) of apatitic structure in which the coefficients are such that :

$0.2 \leqq x \leqq 0.5$
$y$ differs little from $x$
$0.8 \leqq t \leqq 1$
$u < 0.1$

**4.** Biomaterial according to Claim 1, characterised in that it comprises an oxygenated calcium phosphate (ApO) with the following apatitic structure :

$$Ca_{10-x} \square_x (HPO_4)_y (PO_4)_{6-y} (OH)_{2+y-x} \square_{1-y} (O_2)_u$$

wherein

$0 \leqq x \leqq 0.2$
$0 \leqq y \leqq 0.2$
$0.2 \leqq u \leqq 0.8$

**5.** Biomaterial according to one of Claims 1, 2, 3 or 4, characterised in that it comprises a mixture of oxygenated apatitic calcium phosphate (ApO) with the aforementioned formula and a non-oxygenated apatitic calcium phosphate (HAp) with the formula: $Ca_{10} (PO_4)_6 (OH)_2$

**6.** Biomaterial according to one of Claims 1, 2, 3 or 4, characterised in that it comprises a mixture of oxygenated apatitic calcium phosphate (ApO) with the aforementioned formula and a tricalcium phosphate (PTCa) with the formula: $Ca_3 (PO_4)_2$.

**7.** Biomaterial according to one of Claims 1, 2, 3 or 4, characterised in that it comprises a mixture of oxygenated apatitic calcium phosphate (ApO) with the aforementioned formula and calcium carbonate $CaCO_3$ and/or calcium sulphate $CaSO_4$.

**8.** Biomaterial according to one of Claims 1, 2, 3 or 4, characterised in that it comprises a mixture of oxygenated apatitic calcium phosphate (ApO) with the aforementioned formula and a collagen.

**9.** Biomaterial according to Claim 8, characterised in that it comprises a mixture of oxygenated apatitic calcium phosphate (ApO), collagen, glycoaminoglycanes and chondroitin sulphate.

**10.** Biomaterial according to one of Claims 1, 2, 3 or 4, characterised in that it comprises a mixture of oxygenated apatitic calcium phosphate (ApO) with the aforementioned formula and haematological coagulation derivatives, in particular fibrin and fibrinogen.

**11.** Biomaterial according to one of the preceding claims, characterised in that it comprises oxygenated calcium phosphate (ApO) with an apatitic structure in which a fraction of the hydrogen phosphate ions ($HPO_4$) is substituted by carbonate ions ($CO_3$).

**12.** Process for the preparation of a biomaterial according to one of Claims 1, 2, 3 or 4, characterised in that it consists in suspending a calcium phosphate of the following group: tricalcium phosphate (PTCa), dicalcium phosphate (DCPO) or octocalcium phosphate (OCP) in an aqueous solution containing hydrogen peroxide, in letting the suspension develop at a pH-value, at a temperature and for a time depending on the required coefficients x, y, t and u, in physically separating the solid phase from the liquid phase and in drying said solid phase.

13. Process for preparing a biomaterial according to one of Claims 1, 2, 3 or 4, characterised in that it consists in pouring into an aqueous solution containing hydrogen peroxide a soluble calcium salt on the one hand and ammonium phosphate at a pH-value, at a temperature and for a time depending on the required coefficients x, y, t and u on the other hand, and in allowing the precipitate obtained to mature in the parent solution, in physically separating the solid phase from the liquid phase and in drying said solid phase.

14. Process of preparation according to Claim 13, characterised in that the amounts of calcium salt and ammonium phosphate poured into the aqueous hydrogen peroxide solution are such that the atomic ratio Ca/P is between 1.4 and 2.

15. Process of preparation according to one of Claims 12, 13 or 14, characterised in that in the course of development or precipitation the pH-value is set between 5 and 9, said value being the higher the lower the coefficients x and y are required to be within the ranges $0 < x \leqq 1$ and $0 < y \leqq 1$.

16. Process of preparation according to one of Claims 12, 13, 14 or 15, characterised in that the temperature during development or precipitation is set at a value between 20°C and 100°C, said value being the higher the lower the coefficients x, y and t are required to be within the ranges $0 < x \leqq 1$, $0 < y \leqq 1$ and $0 \leqq t \leqq 1$.

17. Process of preparation according to one of Claims 12, 13, 14, 15 or 16, characterised in that the time during which one allows the suspension to develop or during which the precipitation takes place is set to a value between 2 minutes and 300 hours, said value being the higher the lower the coefficients x, y and t are required to be within the ranges $0 \leqq x \leqq 1$, $0 \leqq y \leqq 1$ and $0 \leqq t \leqq 1$.

18. Process of preparation according to one of Claims 12 to 17, characterised in that the aqueous hydrogen peroxide solution is so selected as to be capable of releasing between 5 and 50 times its own volume of oxygen, this volume being the higher the higher the coefficients t and u are required to be within the ranges $0 \leqq t \leqq 1$ and $0 \leqq u \leqq 1$.

19. Process of preparation according to one of Claims 12 to 18, characterised in that it comprises a supplementary stage of calcinating the separated solid phase at a temperature lower than 450°, said temperature being the higher the lower a coefficient t is required to be within the range $0 \leqq t \leqq 1$.

20. Process according to Claim 12 for preparing a biomaterial according to Claim 2 with a reduced proportion of peroxide ions $O^{2-}$, characterised in that once dried the solid phase is calcinated at a temperature between 150° and 300°, said temperature being the higher the lower a coefficient t is required to be.

21. Process according to Claim 13 for preparing a biomaterial according to Claim 3, characterised in that it consists:
    . in preparing an aqueous hydrogen peroxide solution capable of releasing between 25 and 35 times its own volume of oxygen,
    . in pouring into said aqueous hydrogen peroxide solution on the one hand an aqueous solution of calcium nitrate or calcium acetate and on the other hand an aqueous solution of ammonium phosphate so that the atomic ratio Ca/P is between 1.5 and 1.7,
    . in mixing the solutions at a pH-value between 8 and 9, at a temperature between 75°C and 100°C and during a time between 2 and 10 minutes,
    . in separating at the end of the aforementioned time the solid phase and in drying it at a temperature lower than 100°C.

22. Process of preparation according to one of Claims 12 to 21 with a view to obtaining a biomaterial according to one of Claims 5 to 9, characterised in that the dry solid phase is mixed with at least one compound pertaining to the following group, said compound being in divided form or in the form of an aqueous gel: HAp, PTCa, $CaCO_3$, $CaSO_4$, collagen, glycoaminoglycane, chondroitin sulphate.

23. Process of preparation according to one of Claims 12 to 22 with a view to obtaining a biomaterial according to Claim 10, characterised in that potassium carbonate or sodium carbonate is added to the hydrogen peroxide solution.

**Patentansprüche**

1. Biomaterial zur Füllung von Knochen oder Zähnen, mit antiseptischen Eigenschaften zwecks Einschränkung der Proliferation von Mikroorganismen an dem Implantationsort, dadurch gekennzeichnet, daß es mit Sauerstoff angereichertes Calciumphosphat (ApO) mit einer apatitartigen Struktur umfaßt, das Verbindungen aufweist, die zu Oxidationsgraden oxidiert sind, die höher als oder gleich 2- sind, mit der Formel:

$$Ca_{10-x} \square_x (HPO_4)_y (PO_4)_{6-y} (OH)_z (O_2^{2-})_t \square_{2-z-t} (O_2)_u$$

wo $\square$ eine Lücke ist

$0 \leqq x \leqq 1$

$0 \leqq y \leqq 1$

$0 \leqq t \leqq 1$

$0 \leqq u \leqq 1$

$u + t \geqq 0.2$

und $z = 2 + y - x - 2t$

2. Biomaterial nach Anspruch 1, dadurch gekennzeichnet, daß es ein mit Sauerstoff angereichertes Calciumphosphat (ApO) mit apatitartiger Struktur umfaßt, bei dem die Koeffizienten so beschaffen sind, daß

$0,1 \leqq x \leqq 0,4$

y wenig anders ist als x

$t \leqq u/2$

$0,2 \leqq u \leqq 0,8$

3. Biomaterial nach Anspruch 1, dadurch gekennzeichnet, daß es ein mit Sauerstoff angereichertes Calciumphosphat (ApO) mit apatitartiger Struktur umfaßt, deren Koeffizienten so beschaffen sind, daß

$0,2 \leqq x \leqq 0,5$

y wenig anders ist als x

$0,8 \leqq t \leqq 1$

$u < 0, 1$

4. Biomaterial nach Anspruch 1, dadurch gekennzeichnet, daß es ein mit Sauerstoff angereichertes Calciumphosphat (ApO) der folgenden apatitartigen Struktur umfaßt:

$$Ca_{10-x} \square_x (HPO_4)_y (PO_4)_{6-y} (OH)_{2+y-x} \square_{1-y} (O_2)_u$$

wobei

$0 \leqq x \leqq 0,2$

$0 \leqq y \leqq 0,2$

$0,2 \leqq u \leqq 0,8$

5. Biomaterial nach einem der Ansprüche 1, 2, 3 oder 4, dadurch gekennzeichnet, daß es ein Gemisch von mit Sauerstoff angereichertem apatitartigem Calciumphosphat (ApO) der vorstehenden Formel und ein nicht mit Sauerstoff angereichertes apatitartiges Calciumphosphat (HAp) der Formel $Ca_{10}(PO_4)_6(OH)_2$ umfaßt.

6. Biomaterial nach einem der Ansprüche 1, 2, 3 oder 4, dadurch gekennzeichnet, daß es ein Gemisch von mit Sauerstoff angereichertem apatitartigem Calciumphosphat (ApO) der vorstehenden Formel und eines Tricalciumphosphats

(PTCa) der Formel $Ca_3 (PO_4)_2$ umfaßt.

7. Biomaterial nach einem der Ansprüche 1, 2, 3 oder 4, dadurch gekennzeichnet, daß es ein Gemisch von mit Sauerstoff angereichertem apatitartigem Calciumphosphat (ApO) der vorstehenden Formel und Calciumcarbonat ($CaCO_3$ und-/oder Calciumsulfat $CaSO_4$ umfaßt.

8. Biomaterial nach einem der Ansprüche 1, 2, 3 oder 4, dadurch gekennzeichnet, daß es ein Gemisch von mit Sauerstoff angereichertem apatitartigem Calciumphosphat (ApO) der vorstehenden Formel und eines Kollagens umfaßt.

9. Biomaterial nach Anspruch 8, dadurch gekennzeichnet, daß es ein Gemisch von mit Sauerstoff angereichertem apatitartigem Calciumphosphat (ApO), Kollagen, Glykoaminoglykanen und Chondroitinsulfat umfaßt.

10. Biomaterial nach einem der Ansprüche 1, 2, 3 oder 4, dadurch gekennzeichnet, daß es ein Gemisch von mit Sauerstoff angereichertem apatitartigem Calciumphosphat (ApO) der vorstehenden Formel und Blutkoagulationsderivaten, insbesondere Fibrin und Fibrinogen, umfaßt.

11. Biomaterial nach einem der vorstehenden Ansprüche, dadurch gekennzeichnet, daß es mit Sauerstoff angereichertes Calciumphosphat (ApO) apatitartiger Struktur umfaßt, bei der ein Teil der Wasserstoffphosphationen ($HPO_4$) durch Carbonat-Ionen ($CO_3$) substituiert ist.

12. Verfahren zur Herstellung eines Biomaterials nach einem der Ansprüche 1, 2, 3 oder 4, dadurch gekennzeichnet, daß es darin besteht, ein Calciumphosphat der Gruppe Tricalciumphosphat (PTCa), Dicalciumphosphat (DCPO) oder Octocalciumphosphat (OCP) in einer Wasserstoffperoxid enthaltenden wäßrigen Lösung zu suspendieren, die Suspension bei einem pH-Wert, bei einer Temperatur und während einer Zeit entwickeln zu lassen, die von den gewünschten Koeffizienten x, y, t und u abhängen, die feste Phase physikalisch von der flüssigen Phase zu trennen und die besagte feste Phase zu trocknen.

13. Verfahren zur Herstellung eines Biomaterials nach einem der Ansprüche 1, 2, 3 oder 4, dadurch gekennzeichnet, daß es darin besteht, in eine Wasserstoffperoxid enthaltende wäßrige Lösung einerseits ein lösliches Calciumsalz und andererseits Ammoniumphosphat bei einem pH-Wert, bei einer Temperatur und während einer Zeit einzugießen, die von den gewünschten Koeffizienten x, y, t und u abhängen, das gewonnene Präzipitat in der Stammlösung reifen zu lassen, die feste Phase von der flüssigen Phase physikalisch zu trennen und die besagte feste Phase zu trocknen.

14. Herstellungsverfahren nach Anspruch 13, dadurch gekennzeichnet, daß die in die wäßrige Wasserstoffperoxidlösung eingegossenen Mengen von Calciumsalz und Ammoniumphosphat so beschaffen sind, daß das atomare Verhältnis Ca/P zwischen 1,4 und 2 beträgt.

15. Herstellungsverfahren nach einem der Ansprüche 12, 13 oder 14, dadurch gekennzeichnet, daß der pH-Wert während der Entwicklung bzw. Fällung auf einen Wert zwischen 5 und 9 eingestellt wird, der um so höher ist, je niedriger die Koeffizienten x und y innerhalb der Bereiche $0 < x \leqq 1$ und $0 < y \leqq 1$ sein sollen.

16. Herstellungsverfahren nach einem der Ansprüche 12, 13, 14 oder 15, dadurch gekennzeichnet, daß die Temperatur während der Entwicklung bzw. Fällung auf einen Wert zwischen 20°C und 100°C eingestellt wird, der um so höher ist, je niedriger die Koeffizienten x, y und t innerhalb der Bereiche $0 < x \leqq 1$, $0 < y \leqq 1$ und $0 \leqq t \leqq 1$ sein sollen.

17. Herstellungsverfahren nach einem der Ansprüche 12, 13, 14, 15 oder 16, dadurch gekennzeichnet, daß die Zeit während der man die Suspension entwickeln läßt oder während der die Fällung stattfindet auf einen Wert zwischen 2 Minuten und 300 Stunden eingestellt wird, der um so höher ist, je niedriger die Koeffizienten x, y und t innerhalb der Bereiche $0 < x \leqq 1$, $0 \leqq y \leqq 1$ und $0 \leqq t \leqq 1$ sein sollen.

18. Herstellungsverfahren nach einem der Ansprüche 12 bis 17, dadurch gekennzeichnet, daß die Wasserstoffperoxid enthaltende wäßrige Lösung so gewählt wird, daß sie in der Lage ist, Sauerstoff in einem Volumen abzugeben, das 5 bis 50mal größer ist als ihr Eigenvolumen, wobei dieses Volumen um so höher ist als die Koeffizienten t und u innerhalb der Bereiche $0 \leqq t \leqq 1$ und $0 \leqq u \leqq 1$ höher sein sollen.

19. Herstellungsverfahren nach einem der Ansprüche 12 bis 18, dadurch gekennzeichnet, daß es eine zusätzliche Stufe des Kalzinierens der abgeschiedenen festen Phase bei einer Temperatur von weniger als 450° umfaßt, die um so höher ist, je niedriger ein Koeffizient t innerhalb des Bereiches $0 \leqq t \leqq 1$ sein soll.

20. Verfahren nach Anspruch 12 zur Herstellung eines Biomaterials nach Anspruch 2 mit einem verringerten Gehalt an Peroxid-Ionen $O^{2-}$, dadurch gekennzeichnet, daß die einmal getrocknete feste Phase bei einer Temperatur zwischen 150° und 300° kalziniert wird, die um so höher ist, je niedriger ein Koeffizient t sein soll.

21. Verfahren nach Anspruch 13 zur Herstellung eines Biomaterials nach Anspruch 3, dadurch gekennzeichnet, daß es in den folgenden Stufen besteht:
    . im Herstellen einer wäßrigen Wasserstoffperoxidlösung, die in der Lage ist, Sauerstoff in einem Volumen, das 25 bis 35mal so groß ist wie ihr Eigenvolumen, abzugeben,
    . im Eingießen in die besagte wäßrige Wasserstoffperoxidlösung von einerseits einer wäßrigen Nitratlösung oder Calciumacetatlösung und andererseits einer wäßrigen Lösung von Ammoniumphosphat, so daß das atomare Verhältnis Ca/P zwischen 1,5 und 1,7 beträgt,
    . im Mischen der Lösungen bei einem pH-Wert zwischen 8 und 9, bei einer Temperatur zwischen 75°C und 100°C und während einer Zeit von 2 bis 10 Minuten,
    . im Trennen, am Ende der vorstehend genannten Zeit, der festen Phase und im Trocknen dieser Phase bei einer Temperatur von weniger als 100°C.

22. Herstellungsverfahren nach einem der Ansprüche 12 bis 21 zwecks Erzielung eines Biomaterials nach einem der Ansprüche 5 bis 9, dadurch gekennzeichnet, daß die trockene feste Phase mit mindestens einem Bestandteil der folgenden Gruppe in verteilter Form oder in Form eines wäßrigen Gels vermischt wird: HAp, PTCa, $CaCO_3$, $CaSO_4$, Kollagen, Glykoaminoglykan, Chondroitinsulfat.

23. Herstellungsverfahren nach einem der Ansprüche 12 bis 22 zwecks Erzielung eines Biomaterials nach Anspruch 10, dadurch gekennzeichnet, daß man in die Wasserstoffperoxid enthaltende Lösung Kalium- oder Natriumcarbonat einführt.